# EUROPEAN PATENT SPECIFICATION

(11) **EP 3 723 747 B1**
(45) Date of publication and mention of the grant of the patent: **22.11.2023**
(21) Application number: 18911728.6
(22) Date of filing: 14.12.2018
(51) Int. Cl.: A61K 31/192, A61K 31/327, A61K 9/06, A61K 47/32, A61K 9/00, A61K 9/08, A61K 45/06, A61K 47/10, A61P 17/10, A61K 31/164, A61K 31/19, A61K 31/197, A61K 31/728

(54) **THE COMBINATION COMPRISING ADAPALENE AND BENZOYL PEROXIDE**
KOMBINATION MIT ADAPALEN UND BENZOYLPEROXID
ASSOCIATION COMPRENANT DE L'ADAPALÈNE ET DU PEROXYDE DE BENZOYLE

(30) Priority: 15.12.2017 TR 201720506
(43) Date of publication of application: 21.10.2020
(73) Proprietor: Sanovel Ilac Sanayi Ve Ticaret Anonim Sirketi, 34460 Sariyer/Istanbul (TR)
(72) Inventor: TÜRKYILMAZ, Ali, 34460 Istanbul (TR); ZENGINER, Sibel, 34460 Istanbul (TR); ATAMAN, Seval, 34460 Istanbul (TR)
(74) Representative: Sevinç, Erkan
(86) International application number: PCT/TR2018/050811
(87) International publication number: WO 2019/190436

(56) References cited:
- WO-A1-2013/124820
- CN-A- 102 614 194
- US-A1- 2006 014 834
- US-A1- 2011 135 584
- US-A1- 2015 098 918

## Description

### Field of the Invention

The present invention relates to a stable topical composition comprising benzoyl peroxide and adapalene free base or pharmaceutically acceptable salts thereof wherein the composition comprises two phases and pharmaceutically acceptable excipients.

### Background of the Invention

Acne is a disease of the skin in which the pilosebaceous structures of the skin become inflamed, leading to the formation of comedones, pustules, and nodules. It is generally believed that acne arises when hyperkeratosis of the pilosebaceous structure wholly or partially blocks the opening of the structure, resulting in comedones filled with sebum, keratin, and Propionibacterium acnes ("P. acnes"). These lesions are commonly identified as acne. P. acnes naturally occurs in normal skin but is especially and characteristically present in acne lesions. It is believed that metabolic by-products and waste from P. acnes within the pilosebaceous structures cause or contribute to the inflammation of acne lesions. Conventional acne treatments have been available as oral and topical dosage forms. Oral drugs include antibiotics like tetracycline, minocycline, doxycycline, and erythromycin. Topical agents for the treatment of acne include retinoids such as tretinoin and adapalene; sulfur; resorcinol; salicylic acid; benzoyl peroxide; and antibiotics such as erythromycin, clindamycin, or tetracycline.

The chemical name of adapalene is 6- [3- (1-Adamantyl) -4-methoxyphenyl] -2-naphthoic acid. Its chemical structure, shown below (Formula I), has a formula of C₂₈H₂₈O₃, with the molecular weight of 412.529 g/mol.

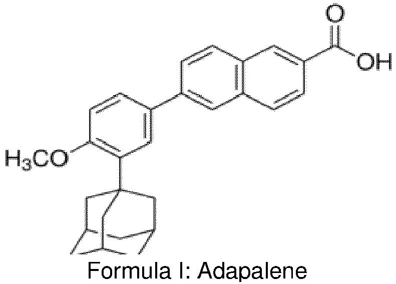

Adapalene is a naphthoic acid derivative with retinoid and anti-inflammatory properties. This molecule was the subject of development for the topical treatment of common acne and of dermatoses sensitive to retinoids.

Adapalene is sold under the brand name Differin^{®} at a weight concentration of 0.1%, in the form of an "alcoholic lotion" solution, an aqueous gel and a cream. These compositions are intended for treating acne. Patent application FR2837101 describes adapalene compositions at a weight concentration of 0.3%, for treating acne.

Benzoyl peroxide, the chemical structure shown below (Formula II), has a formula of C₁₄H₁₀O₄ with the molecular weight of 242.23 g/mol:

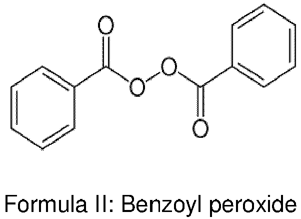

Benzoyl peroxide ("BPO") has been widely used for the treatment of acne. Gel or cream containing benzoyl peroxide is usually rubbed into the pores over the affected region. In addition to its therapeutic effect as a keratolytic (a chemical that dissolves the keratin plugging the pores), benzoyl peroxide also prevents new lesions by killing P. acnes. Benzoyl peroxide has the advantage of being a strong oxidizer and thus does not appear to generate bacterial resistance.

Patent application WO 03/055472 moreover describes stable pharmaceutical compositions comprising adapalene and benzoyl peroxide (BPO).

In the prior art, there are many patent applications for topical formulations comprising adapalene and benzoyl peroxide. However, formulating such a composition poses several problems because of properties of active substances.

There is thus still a need for a physically and chemically stable composition containing benzoyl peroxide and adapalene. So as to minimize interaction of benzoyl peroxide with adapalene and enhance stability of composition, placing drug substances in separate phases is an easy and cheap way.

US 2006/014834 A1 discloses an apparatus for applying a topical composition onto the skin.

The apparatus comprises two different chambers wherein one of the chambers contains an aqueous phase and the other chamber contains a non-aqueous phase. The stability problem is solved by preventing the direct contact of retinoids with other components during storage period. This is ensured by a chamber-in-chamber dispenser which provides the retinoid component and benzoyl peroxide in separate chambers.

### Detailed Description of the Invention

The main object of the present invention is to provide improved compositions which are less irritating, are easy to formulate, have a smooth consistency after formulation, are adequately stable and have a sufficiently long storage life.

Another object of the present invention is to provide rapid penetration of drug substances with minimized skin irritation.

The term "gelling agent", as used herein, is synonymous with viscosity agent, solidifier, or thickening agent, and refers to an agent that increases the viscosity of the formulation by forming a polymeric structure.

Firstly, the efficacy of benzoyl peroxide is associated with its decomposition when it is placed in contact with the skin. When benzoyl peroxide penetrates the skin, it is metabolized by the amino acid cysteine into benzoic acid and an oxygen free radical. Specifically, the efficacy comes from the oxidizing properties of the free radicals produced during this decomposition that lead to the desired effect. Oxygen free radical released by benzoyl peroxide reacts with cell wall proteins of the bacterium, hence unfolding it and destroying the bacterium. Thus, in order to maintain the optimum efficacy of benzoyl peroxide, it is important to prevent its decomposition before use, i.e., during storage.

Benzoyl peroxide is an unstable chemical compound. Degradation of benzoyl peroxide results in formation of benzoic acid impurity that affects the stability of not only excipients used but also other active substances. Separating phases of benzoyl peroxide and adapalene as the water and the oil phases is an effective way to decrease undesired effects of benzoyl peroxide upon adapalene.

According to an embodiment of the present invention, benzoyl peroxide is in the water phase in order that benzoyl peroxide is more stable in water.

According to an embodiment of the present invention, stabilization of adapalene by dispersing in the oil phase has been proposed. Adapalene is insoluble in water. It has been found that mixing adapalene with light liquid paraffin and then dispersing in soft paraffin is very effective way to maintain homogeneous dispersion and desired consistency in the oil phase.

According to an embodiment of the present invention, a stable topical composition comprises
- A water phase comprising benzoyl peroxide
- An oil phase comprising adapalene free base or pharmaceutically acceptable salts of adapalene wherein the weight ratio of the oil phase to the water phase is between 0.5 and 1.5.

According to one embodiment of this invention, the weight ratio of the oil phase to the wate phase is between 0.75 - 1.20 or 0.90 - 1.10.

According to one embodiment of this invention, the weight ratio of the water phase to the tota composition is between 28.0% and 69.0%, 35.0% and 55.0%. It may be between 28.0% and 30.0%, 30.0% and 40.0%, 40.0% and 55.0%, 55.0% and 62.0%, 62.0% and 69.0%.

According to one embodiment of this invention, the weight ratio of the oil phase to the total composition is between 31.0% and 72.0%, 45.0% and 65.0%. It may be between 31.0% and 38.0%, 38.0% and 45.0%, 45.0% and 55.0%, 55.0% and 65.0%, 65.0% and 72.0%.

According to another embodiment of the present invention, more preferably, the weight ratio of the water phase to the total composition is 50.0%, the weight ratio of the oil phase to the total composition is 50.0%. More preferably, the weight ratio of the oil phase to the water phase is 1:1.

According to another embodiment of the present invention, the stable topical composition comprises,
- A water phase comprising benzoyl peroxide dispersed in propylene glycol
- An oil phase comprising adapalene free base or pharmaceutically acceptable salts of adapalene wherein the weight ratio of the oil phase to the water phase is between 0.5 - 1.5, 0.75 - 1.20, 0.90 - 1.10, preferably the weight ratio is 1.0 -1.0. So, this formulation provides long-term stability and prevents degradation of active agents.

According to one embodiment of this invention, the composition comprises pharmaceutically acceptable excipients which are selected from the group comprising gelling agents, oil phase agents, humectants, solvents, co-solvents, preservatives, antioxidants, surfactants, pH-adjusting agents, perfumes or mixtures thereof.

Suitable gelling agents are selected from the group comprising carbomer, liquid paraffin, polyacrylamide, cellulosic polymers such as hydroxypropyl cellulose, hydroxyethyl cellulose, methyl cellulose, and hydroxypropylmethyl cellulose, modified starch, acrylic acid/ethyl acrylate copolymers, polymethacrylate copolymers, trihydroxystearin, aluminum magnesium hydroxy stearate, poloxamer, polyvinyl alcohol, polyvinyl pyrrolidone, methyl hydroxybenzoate, ethyl hydroxybenzoate, propyl hydroxybenzoate, butyl hydroxybenzoate or mixtures thereof.

According to this embodiment of the invention, the gelling agents are between 0.01% and 8.0% by weight of the total composition.

At this invention, carbomer (carbomer 940) is used as gelling agent, because it is highly efficient thickener and it is ideal for formulating clear aqueous and gel. Its benefits include short flow properties, high viscosity, high suspending ability, and high clarity.

According to one embodiment of the invention, carbomer (carbomer 940) is between 0.01% and 8.0% by weight of the total composition.

The stable topical composition in the present invention has a viscosity in a range of between 5 Pa.s. and 40 Pa.s at room temperature (20°) and preferably between 8 Pa.s and 25 Pa.s. The viscosity of the final composition is arranged to provide a desired topical composition consistency. The composition should be pleased by the patient. The viscosity is measured by using RVDV-II or LVDV-II [GR1] Brookfield Digital Viscometers in the conditions of room temperature and 20 rpm. Time of stabilization is 30 seconds.

Suitable oil phase agents are selected from the group comprising soft paraffin, liquid paraffin, stearyl alcohol, ethyl oleate, almond oil, palm oil, sesame oil, oil sunflower, lanolin, squalene, mink oil, cetearyl isononanoate, diisopropyl adipate, isopropyl palmitate, caprylic/capric triglyceride, an oil volatile or non-volatile silicone or mixtures thereof.

According to this embodiment of the invention, the oil phase agents are between 12.00% and 54.0% by weight of the total composition.

According to one embodiment of the present invention, the oil phase agent is soft paraffin which is between 12.00% and 54.0% by weight of the total composition.

A humectant is a hygroscopic substance used to keep things moist. A humectant attracts and retains the moisture in the air nearby via absorption, drawing the water vapor into and/or beneath the organism/object's surface.

Humectants can be used in topical dosage forms to increase the solubility of a chemical compound's active ingredients, increasing the active ingredients' ability to penetrate skin, and/or its activity time.

Suitable humectants are selected from the group comprising propylene glycol, glycerin, butylene glycol, urea, tremella extract, sorbitol, dicyanamide, sodium lactate or mixtures thereof.

According to this embodiment of the invention, the humectants are between 0.3% and 10.0% by weight of the total composition.

Suitable solvents and co-solvents are selected from the group comprising water, propylene glycol, glycerin, ethanol, polyethylene glycol or mixtures thereof.

According to this embodiment of the invention, the solvents and co-solvents are between 26.0% and 60.0% by weight of the total composition.

Also, benzoyl peroxide is more stable in water and propylene glycol when it is in dispersed form. Thus, in order to limit the problem of rapid instability of benzoyl peroxide in solution, benzoyl peroxide in the water phase is formulated with propylene glycol in dispersed form.

Suitable antimicrobial preservatives are selected from the group comprising citric acid, methyl, ethyl, propyl, and butyl esters of hydroxy benzoic acid, benzoic acid, boric acid, chlorhexidine, benzalkonium chloride, 2-phenoxyethanol, cetrimide, potassium sorbate, thiomersal, or mixtures thereof.

The antioxidant is used in the composition to retard oxidation and decomposition of the active ingredients and other substances, thus providing the composition with long-term stability.

Suitable antioxidants are selected from the group comprising ascorbyl palmitate, tocopherol, citric acid, butylated hydroxyanisole (BHA), butylated hydroxytoluene (BHT), sodium metabisulfite, ascorbic acid, thiourea, acetylcysteine, dithiothreitol, cysteine hydrochloride, propyl gallate, vitamin E polyethylene glycol succinate, malic acid, sodium sulfite, rutoside, biotin, Pyridoxine, coniferyl alcohol, oxidized glutathione, pyrocatechine, butylparaben, propylparaben, dry green tea extract, resveratrol, quercetin, ubiquinone, pyridoxine, acetyl pantothenate, beta-ionol, 8-hydroxyquinoline, hydroquinone, creatinine, naphthoquinone, turmeric extract (curcumin) or superoxide dismutase or mixtures thereof.

A surfactant may also be included in the formulation of the present invention to ensure homogeneous dispersion of hydrophilic and hydrophobic phases in the composition. Suitable surfactants are selected from the group comprising propylene glycol, glyceryl oleate, tocopherol, ascorbyl palmitate, citric acid, polyethoxylated fatty acid esters, polyoxyethylene sorbitan esters, polyoxyethylene hydrogenated castor oil, sorbitan esters, sodium lauryl sulphate, docusate sodium, nonoxynol or mixtures thereof.

According to this embodiment of the invention, the surfactants are between 0.01% and 10.0% by weight of the total composition.

The pharmaceutical composition of the present invention may also include one or more pH-adjusting agents. Suitable pH-adjusting agents are selected from the group comprising pharmaceutically acceptable organic or inorganic acids or bases, for example, sodium hydroxide, tromethamine, hydrochloric acid or mixtures thereof. Particularly, the compositions of the present invention have a pH of about 5.0 to about 6.0.

Suitable perfumes are selected from the group comprising lavender oil, rose oil, lemon oil, and almond oil.

The stable topical composition of the present invention is present in the form of a gel, solution, foam, cream, pomade, lotion, or spray. More particularly, it is in the form of a gel.

According to one embodiment of this invention, the composition further may comprise adapalene free base or pharmaceutically acceptable salts thereof, benzoyl peroxide and an active agent from the cicatrizant group.

Suitable active agents from the cicatrizant group are selected from the group comprising sodium hyaluronate, enoxolone, hyaluronic acid, calcium pantothenate, pantothenic acid, tetrachloro decaoxide, kadexomer iodide, dextranomer, dexpanthenol or mixtures thereof, preferably the cicatrizant is sodium hyaluronate.

In one embodiment of the invention, the composition comprises;
- Water phase
   a) 0.05 - 20.0% by weight of benzoyl peroxide
   b) 0.01 - 8.0% by weight of carbomer
   c) 0.5 - 5.0% by weight of propylene glycol
   d) 0.5 - 5.0% by weight of glycerin
   e) 0.0001 - 5.0% by weight of sodium hydroxide solution (20 g NaOH/100 ml water)
   f) 26.0 - 60.0% by weight of purified water
- Oil phase
   a) 0.001-20.0% by weight of adapalene free base or pharmaceutically acceptable salts thereof
   b) 5.0-40.0% by weight of soft paraffin
   c) 5.0-40.0% by weight of liquid paraffin
   d) 0.001-5.0% by weight of propylene glycol, glyceryl oleate, tocopherol, ascorbyl palmitate and citric acid mixture

The process for the preparation of the stable topical composition comprises the following steps:
- Water phase
   a) Mixing enough water and carbomer until obtaining homogeneous mixture
   b) Adding sodium hydroxide solution and then mixing
   c) Dispersing benzoyl peroxide in propylene glycol on a separate tank
   d) Adding glycerin to step(c) mixture and then mixing and homogenizing
   e) Adding step(d) mixture to step(b) mixture and then adjusting pH value 5.0-6.0
- Oil phase
   a) Mixing liquid paraffin and adapalene free base or pharmaceutically acceptable salts thereof
   b) Adding soft paraffin and mixing until no phase separation
   c) Adding propylene glycol, glyceryl oleate, tocopherol, ascorbyl palmitate, citric acid mixture and mixing until obtaining homogenous mixture.

The oil phase is added to the water phase and mixed until homogenious.

So as to ensure homogeneity of not only dispersed active substances but also distribution of phases, homogenizer is used at each step of the process.

### Example 1

| **Water phase** | **amount (% by weight of the total)** |
|---|---|
| Benzoyl peroxide | 0.08%-12.0% |
| Carbomer | 0.05%-6.0% |
| Propylene glycol | 0.7%-4.0% |
| Glycerin | 0.7%-4.0% |
| Sodium hydroxide solution (20 g NaOH/100 ml water) | 0.001%-3.0% |
| Purified water | 35%-50.0% |

| **Oil phase** | **amount (% by weight of the total)** |
|---|---|
| Adapalene free base or pharmaceutically acceptable salts of adapalene | 0.08%-12.0% |
| Soft paraffin | 8.0%-35.0% |
| Liquid paraffin | 8.0%-35.0% |
| Propylene glycol, glyceryl oleate, tocopherol, ascorbyl palmitate and citric acid mixture | 0.005%-4.0% |
| **Total composition** | **100** |

According to example 1, the weight ratio of the water phase to the total composition may be between 55.0% and 69.0%, the weight ratio of the oil phase to the total composition may be between 31.0% and 45.0%.

### Example 2

| **Water phase** | **amount (% by weight of the total)** |
|---|---|
| Benzoyl peroxide | 0.05%-20.0% |
| Carbomer | 0.01%-8.0% |
| Propylene glycol | 0.5%-5.0% |
| Glycerin | 0.5%-5.0% |
| Sodium hydroxide solution (20 g NaOH/100 ml water) | 0.0001%-5.0% |
| Purified water | 26.0%-60.0% |

| **Oil phase** | **amount (% by weight of the total)** |
|---|---|
| Adapalene free base or pharmaceutically acceptable salts of adapalene | 0.001%-20.0% |
| Soft paraffin | 5.0%-40.0% |
| Liquid paraffin | 5.0%-40.0% |
| Propylene glycol, glyceryl oleate, tocopherol, ascorbyl palmitate and citric acid mixture | 0.001%-5.0% |
| **Total composition** | **100** |

According to example 2, the weight ratio of the water phase to the total composition may be between 40.0% and 55.0%, the weight ratio of the oil phase to the total composition may be between 45.0% and 60.0%. More preferably, this the weight ratio of the water phase to the total composition is 50.0%, the weight ratio of the oil phase to the total composition is 50.0%

### Example 3

| **Water phase** | **amount (% by weight of the total)** |
|---|---|
| Benzoyl peroxide | 0.08%-12.0% |
| Carbomer 940 | 0.05%-6.0% |
| propylene glycol | 0.7%-4.0% |
| glycerin | 0.7%-4.0% |
| sodium hydroxide solution (20 g NaOH/100 ml water) | 0.001%-3.0% |
| purified water | 35%-50.0% |

| **Oil phase** | **amount (% by weight of the total)** |
|---|---|
| Adapalene free base or pharmaceutically acceptable salts of adapalene | 0.08%-12.0% |
| soft paraffin | 8.0%-35.0% |
| liquid paraffin | 8.0%-35.0% |
| propylene glycol, glyceryl oleate, tocopherol, ascorbyl palmitate and citric acid mixture | 0.005%-4.0% |
| **Total composition** | **100** |

According to example 3, the weight ratio of the water phase to the total composition may be between 28.0% and 40.0%, the weight ratio of the oil phase to the total composition may be between 60.0% and 72.0%.

### Process for example 1, 2 and 3

The process for the preparation of the stable topical composition comprises the following steps:
- Water phase
   a) Mixing enough water and carbomer 940 until obtaining homogeneous mixture
   b) Adding sodium hydroxide solution and then mixing
   c) Dispersing benzoyl peroxide in propylene glycol on a separate tank
   d) Adding glycerin to step(c) mixture and then mixing and homogenizing
   e) Adding step(d) mixture to step(b) mixture and then adjusting pH value 5.0-6.0
- Oil phase
   a) Mixing liquid paraffin and adapalene free base or pharmaceutically acceptable salts thereof
   b) Adding soft paraffin and mixing until no phase separation
   c) Adding Cithrol GMO 50-LQ-(RB) and mixing until obtaining homogeneous mixture

The oil phase is added in the water phase and mixed until homogenious.

### Example 4

| **Water phase** | **amount (% by weight of the total)** |
|---|---|
| Benzoyl peroxide | 0.05%-20.0% |
| Sodium hyaluronate | 0.05%-20.0% |
| Pharmaceutically acceptable excipients | 1.0%-20.0% |
| Purified water | 20.0%-50.0% |

| **Oil phase** | **amount (% by weight of the total)** |
|---|---|
| Adapalene free base or pharmaceutically acceptable salts of adapalene | 0.05%-20.0% |
| The oil phase agent | 5.0%-54.0% |
| Pharmaceutically acceptable excipients | 1.0%-20.0% |
| **Total composition** | **100** |

### Example 5

| **Water phase** | **amount (% by weight of the total)** |
|---|---|
| Benzoyl peroxide | 0.05%-20.0% |
| Pharmaceutically acceptable excipients | 1.0%-20.0% |
| Purified water | 20.0%-50.0% |

| **Oil phase** | **amount (% by weight of the total)** |
|---|---|
| Adapalene free base or pharmaceutically acceptable salts of adapalene | 0.05%-20.0% |
| Sodium hyaluronate | 0.05%-20.0% |
| The oil phase agent | 5.0%-54.0% |
| Pharmaceutically acceptable excipients | 1.0%-20.0% |
| **Total composition** | **100** |

## Claims

1. A stable topical composition comprising;
- A water phase comprising benzoyl peroxide
- An oil phase comprising adapalene free base or pharmaceutically acceptable salts of adapalene wherein the weight ratio of the oil phase to the water phase is between 0.5 and 1.5.

2. The stable topical composition according to claim 1, wherein the weight ratio of the water phase to the total composition is between 28.0% and 69.0%.

3. The stable topical composition according to claim 1, wherein the weight ratio of the oil phase to the total composition is between 31.0% and 72.0%.

4. The stable topical composition according to any preceding claims, wherein the composition further comprising pharmaceutically acceptable excipients which are selected from the group comprising gelling agents, oil phase agents, humectants, solvents, co-solvents, preservatives, antioxidants, surfactants, pH-adjusting agents, perfumes or mixtures thereof.

5. The stable topical composition according to claim 4, wherein gelling agents are selected from the group comprising carbomer, liquid paraffin, polyacrylamide, hydroxypropyl cellulose, hydroxyethyl cellulose, methyl cellulose, and hydroxypropylmethyl cellulose, modified starch, acrylic acid/ethyl acrylate copolymers, polymethacrylate copolymers, trihydroxystearin, aluminum magnesium hydroxy stearate, poloxamer, polyvinyl alcohol, polyvinyl pyrrolidone, methyl hydroxybenzoate, ethyl hydroxybenzoate, propyl hydroxybenzoate, butyl hydroxybenzoate or mixtures thereof.

6. The stable topical composition according to claim 5, wherein the gelling agent is carbomer.

7. The stable topical composition according to claim 4, wherein oil phase agents are selected from the group comprising soft paraffin, liquid paraffin, stearyl alcohol, ethyl oleate, almond oil, palm oil, sesame oil, oil sunflower, lanolin, squalene, mink oil, cetearyl isononanoate, diisopropyl adipate, isopropyl palmitate, caprylic/capric triglyceride, a volatile or non-volatile silicone or mixtures thereof.

8. The stable topical composition according to claim 7, wherein the oil phase agent is soft paraffin.

9. The stable topical composition according to any preceding claims, wherein the composition is present in the form of a gel, solution, foam, lotion, cream, pomade or spray.

10. The stable topical composition according to claim 9, wherein the composition is present in the form of a gel.

11. The stable topical composition according to any preceding claims, wherein the composition further comprising one more active agent which is selected from the cicatrizant group.

12. The stable topical composition according to claim 11, wherein the active agent from the cicatrizant group are selected from the group comprising sodium hyaluronate, enoxolone, hyaluronic acid, calcium pantothenate, pantothenic acid, tetrachloro decaoxide, kadexomer iodide, dextranomer, dexpanthenol or mixtures thereof.

13. The stable topical composition according to any preceding claims, the composition comprising;
• Water phase
a) 0.05 - 20.0% by weight of benzoyl peroxide
b) 0.01 - 8.0% by weight of carbomer
c) 0.5 - 5.0% by weight of propylene glycol
d) 0.5 - 5.0% by weight of glycerin
e) 0.0001 - 5.0% by weight of sodium hydroxide solution (20 g NaOH/100 ml water)
f) 26.0 - 60.0% by weight of purified water
• Oil phase
a) 0.001-20.0% by weight of adapalene free base or pharmaceutically acceptable salts thereof
b) 5.0-40.0% by weight of soft paraffin
c) 5.0-40.0% by weight of liquid paraffin
d) 0.001-5.0% by weight of propylene glycol, glyceryl oleate, tocopherol, ascorbyl palmitate and citric acid

14. The process for the preparation of the stable topical composition according claim 13, wherein the process comprising the following steps:
• Water phase
a) Mixing enough water and carbomer until obtaining homogeneous mixture
b) Adding sodium hydroxide solution and then mixing
c) Dispersing benzoyl peroxide in propylene glycol on a separate tank
d) Adding glycerin at step(c) mixture and then mixing until homogenious
e) Adding step(d) mixture to step(b) mixture and then adjusting pH value between 5.0 and 6.0.
• Oil phase
a) Mixing liquid paraffin and adapalene free base or pharmaceutically acceptable salts thereof
b) Adding soft paraffin and mixing until no phase separation
c) Adding propylene glycol, glyceryl oleate, tocopherol, ascorbyl palmitate, citric acid mixture and mixing until obtaining homogeneous mixture
The oil phase is added in the water phase and mixed until obtaining homogeneous mixture.

## Patentansprüche

1. Stabile topische Zusammensetzung, aufweisend:
- eine Wasserphase mit Benzoylperoxid,
- eine Ölphase, die eine Adapalen-freie Base oder pharmazeutisch annehmbare Salze von Adapalen enthält, wobei das Gewichtsverhältnis der Ölphase zu der Wasserphase zwischen 0,5 und 1,5 liegt.

2. Stabile topische Zusammensetzung gemäß Anspruch 1, wobei das Gewichtsverhältnis der Wasserphase zu der Gesamtzusammensetzung zwischen 28,0 % und 69,0 % liegt.

3. Stabile topische Zusammensetzung gemäß Anspruch 1, wobei das Gewichtsverhältnis der Ölphase zu der Gesamtzusammensetzung zwischen 31,0 % und 72,0 % liegt.

4. Stabile topische Zusammensetzung nach einem der vorstehenden Ansprüche, wobei die Zusammensetzung ferner pharmazeutisch annehmbare Hilfsstoffe enthält, die aus der Gruppe ausgewählt sind, die Geliermittel, Ölphasenmittel, Feuchthaltemittel, Lösungsmittel, Co-Lösungsmittel, Konservierungsmittel, Antioxidantien, Tenside, pH-Einstellmittel, Duftstoffe oder Mischungen davon umfasst.

5. Stabile topische Zusammensetzung gemäß Anspruch 4, wobei Geliermittel aus der Gruppe ausgewählt sind, die Carbomer, flüssiges Paraffin, Polyacrylamid, Hydroxypropylcellulose, Hydroxyethylcellulose, Methylcellulose und Hydroxypropylmethylcellulose, modifizierte Stärke, Acrylsäure/Ethylacrylat-Copolymere, Polymethacrylat-Copolymere, Trihydroxystearin, Aluminiummagnesiumhydroxystearat, Poloxamer, Polyvinylalkohol, Polyvinylpyrrolidon, Methylhydroxybenzoat, Ethylhydroxybenzoat, Propylhydroxybenzoat, Butylhydroxybenzoat oder Mischungen davon aufweist.

6. Stabile topische Zusammensetzung gemäß Anspruch 5, wobei das Geliermittel Carbomer ist.

7. Stabile topische Zusammensetzung gemäß Anspruch 4, wobei die Ölphasenmittel aus der Gruppe ausgewählt sind, die Weichparaffin, flüssiges Paraffin, Stearylalkohol, Ethyloleat, Mandelöl, Palmöl, Sesamöl, Sonnenblumenöl, Lanolin, Squalen, Nerzöl, Cetearylisononanoat, Diisopropyladipat, Isopropylpalmitat, Caprylic/Caprinsäuretriglycerid, ein flüchtiges oder nichtflüchtiges Silikon oder Mischungen davon aufweist.

8. Stabile topische Zusammensetzung gemäß Anspruch 7, wobei das Ölphasenmittel Weichparaffin ist.

9. Stabile topische Zusammensetzung gemäß einem der vorstehenden Ansprüche, wobei die Zusammensetzung in Form eines Gels, einer Lösung, eines Schaums, einer Lotion, einer Creme, einer Pomade oder eines Sprays vorliegt.

10. Stabile topische Zusammensetzung gemäß Anspruch 9, wobei die Zusammensetzung in Form eines Gels vorliegt.

11. Stabile topische Zusammensetzung gemäß einem der vorstehenden Ansprüche, wobei die Zusammensetzung ferner einen weiteren Wirkstoff enthält, der aus der Gruppe der Vernarbungsmittel ausgewählt ist.

12. Stabile topische Zusammensetzung gemäß Anspruch 11, wobei der Wirkstoff aus der Gruppe der Vernarbungsmittel aus der Gruppe ausgewählt ist, die Natriumhyaluronat, Enoxolon, Hyaluronsäure, Calciumpantothenat, Pantothensäure, Tetrachlordekaoxid, Kadexomerjodid, Dextranomer, Dexpanthenol oder Mischungen davon umfasst.

13. Stabile topische Zusammensetzung gemäß einem der vorstehenden Ansprüche, wobei die Zusammensetzung umfasst:
• Wasserphase
a) 0,05 - 20,0 Gew.-% Benzoylperoxid
b) 0,01 - 8,0 Gew.-% Carbomer
c) 0,5 - 5,0 Gew.-% Propylenglykol
d) 0,5 - 5,0 Gew.-% Glycerin
e) 0,0001 - 5,0 Gew.-% Natriumhydroxidlösung (20 g NaOH/100 ml Wasser)
f) 26,0 - 60,0 Gew.-% gereinigtes Wasser
- Ölphase
a) 0,001 - 20,0 Gew.-% freie Adapalen-Base oder pharmazeutisch annehmbare Salze davon
b) 5,0 - 40,0 Gew.-% Weichparaffin
c) 5,0 - 40,0 Gew.-% flüssiges Paraffin
d) 0,001 - 5,0 Gew.-% Propylenglykol, Glycerinoleat, Tocopherol, Ascorbylpalmitat und Zitronensäure

14. Verfahren zur Herstellung der stabilen topischen Zusammensetzung gemäß Anspruch 13, wobei das Verfahren die folgenden Schritte umfasst:
• Wasserphase
a) Mischen von ausreichend Wasser und Carbomer, bis eine homogene Mischung erhalten wird,
b) Zugeben von Natriumhydroxidlösung und anschließendes Mischen,
c) Dispergieren von Benzoylperoxid in Propylenglykol in einem separaten Gefäß,
d) Zugeben von Glycerin in Schritt c) und anschließendes Mischen, bis die Mischung homogen ist,
e) Zugeben der Mischung aus Schritt (d) zu der Mischung aus Schritt (b) und anschließende Einstellung des pH-Werts zwischen 5,0 und 6,0.
• Ölphase
a) Mischen von flüssigem Paraffin und Adapalen-freier Base oder deren pharmazeutisch annehmbaren Salzen,
b) Zugeben von weichem Paraffin und Mischen, bis keine Phasentrennung mehr vorliegt,
c) Zugeben von Propylenglykol, Glycerinoleat, Tocopherol, Ascorbylpalmitat und Zitronensäure und Mischen, bis ein homogenes Gemisch erhalten wird
Die Ölphase wird in die Wasserphase gegeben und gemischt, bis eine homogene Mischung erhalten wird.

## Revendications

1. - Composition topique stable comprenant :
- une phase aqueuse comprenant du peroxyde de benzoyle ;
- une phase huileuse comprenant de l'adapalène base libre ou des sels pharmaceutiquement acceptables d'adapalène,
- le rapport en poids de la phase huileuse à la phase aqueuse étant entre 0,5 et 1,5.

2. - Composition topique stable selon la revendication 1, dans laquelle le rapport en poids de la phase aqueuse à la composition totale est entre 28,0 % et 69,0 %.

3. - Composition topique stable selon la revendication 1, dans laquelle le rapport en poids de la phase huileuse à la composition totale est entre 31,0 % et 72,0 %.

4. - Composition topique stable selon l'une quelconque des revendications précédentes, dans laquelle la composition comprend en outre des excipients pharmaceutiquement acceptables qui sont choisis dans le groupe comprenant les agents gélifiants, les agents de phase huileuse, les humectants, les solvants, les co-solvants, les conservateurs, les antioxydants, les tensioactifs, les agents d'ajustement du pH, les parfums ou les mélanges de ceux-ci.

5. - Composition topique stable selon la revendication 4, dans laquelle les agents gélifiants sont choisis dans le groupe comprenant les carbomères, la paraffine liquide, le polyacrylamide, l'hydroxypropyl cellulose, l'hydroxyéthyl cellulose, la méthyl cellulose et l'hydroxypropylméthyl cellulose, l'amidon modifié, les copolymères acide acrylique/acrylate d'éthyle, les copolymères de polyméthacrylate, la trihydroxystéarine, l'hydroxy stéarate d'aluminium et de magnésium, les poloxamères, l'alcool polyvinylique, la polyvinyl pyrrolidone, l'hydroxybenzoate de méthyle, l'hydroxybenzoate d'éthyle, l'hydroxybenzoate de propyle, l'hydroxybenzoate de butyle ou les mélanges de ceux-ci.

6. - Composition topique stable selon la revendication 5, dans laquelle l'agent gélifiant est un carbomère.

7. - Composition topique stable selon la revendication 4, dans laquelle les agents de phase huileuse sont choisis dans le groupe comprenant la paraffine molle, la paraffine liquide, l'alcool stéarylique, l'oléate d'éthyle, l'huile d'amande, l'huile de palme, l'huile de sésame, l'huile de tournesol, la lanoline, le squalène, l'huile de vison, l'isononanoate de cétéaryle, l'adipate de diisopropyle, le palmitate d'isopropyle, le triglycéride caprylique/caprique, un silicone volatil ou non volatil ou les mélanges de ceux-ci.

8. - Composition topique stable selon la revendication 7, dans laquelle l'agent de phase huileuse est la paraffine molle.

9. - Composition topique stable selon l'une quelconque des revendications précédentes, dans laquelle la composition est présente sous la forme d'un gel, d'une solution, d'une mousse, d'une lotion, d'une crème, d'une pommade ou d'un spray.

10. - Composition topique stable selon la revendication 9, dans laquelle la composition est présente sous la forme d'un gel.

11. - Composition topique stable selon l'une quelconque des revendications précédentes, dans laquelle la composition comprend en outre un agent actif supplémentaire qui est choisi dans le groupe des cicatrisants.

12. - Composition topique stable selon la revendication 11, dans laquelle l'agent actif du groupe des cicatrisants est choisi dans le groupe comprenant l'hyaluronate de sodium, l'énoxolone, l'acide hyaluronique, le pantothénate de calcium, l'acide pantothénique, le tétrachloro-décaoxyde, l'iodure de cadexomère, le dextranomère, le dexpanthénol ou les mélanges de ceux-ci.

13. - Composition topique stable selon l'une quelconque des revendications précédentes, la composition comprenant :
• Phase aqueuse
a) 0,05 à 20,0 % en poids de peroxyde de benzoyle
b) 0,01 à 8,0 % en poids de carbomère
c) 0,5 à 5,0 % en poids de propylène glycol
d) 0,5 à 5,0 % en poids de glycérine
e) 0,0001 à 5,0 % en poids de solution d'hydroxyde de sodium (20 g NaOH/100 ml d'eau)
f) 26,0 à 60,0 % en poids d'eau purifiée
• Phase huileuse
a) 0,001 à 20,0 % en poids d'adapalène base libre ou de sels pharmaceutiquement acceptables de celle-ci
b) 5,0 à 40,0 % en poids de paraffine molle
c) 5,0 à 40,0 % en poids de paraffine liquide
d) 0,001 à 5,0 % en poids de propylène glycol, d'oléate de glycéryle, de tocophérol, de palmitate d'ascorbyle et d'acide citrique.

14. - Procédé de préparation de la composition topique stable selon la revendication 13, dans lequel le procédé comprend les étapes suivantes :
• Phase aqueuse
a) Mélanger suffisamment d'eau et le carbomère jusqu'à l'obtention d'un mélange homogène
b) Ajouter la solution d'hydroxyde de sodium puis mélanger
c) Disperser le peroxyde de benzoyle dans le propylène glycol sur une cuve séparée
d) Ajouter la glycérine au mélange de l'étape (c) puis mélanger jusqu'à homogénéité
e) Ajouter le mélange de l'étape (d) au mélange de l'étape (b), puis ajuster la valeur du pH entre 5,0 et 6,0.
• Phase huileuse
a) Mélanger la paraffine liquide et l'adapalène base libre ou les sels pharmaceutiquement acceptables de celle-ci
b) Ajouter la paraffine molle et mélanger jusqu'à ce qu'il n'y ait plus de séparation de phases
c) Ajouter le mélange de propylène glycol, d'oléate de glycéryle, de tocophérol, de palmitate d'ascorbyle, d'acide citrique et mélanger jusqu'à obtention d'un mélange homogène,
la phase huileuse est ajoutée dans la phase aqueuse et mélangée jusqu'à obtention d'un mélange homogène.
